# EUROPEAN PATENT SPECIFICATION

(11) **EP 2 879 783 B1**
(45) Date of publication and mention of the grant of the patent: **05.09.2018**
(21) Application number: 13777132.5
(22) Date of filing: 30.07.2013
(51) Int. Cl.: B01F 17/52, B82Y 5/00

(54) **METHOD FOR OBTAINING ULTRASTABLE NANOEMULSIONS**
VERFAHREN ZUR GEWINNUNG VON ULTRASTABILEN NANOEMULSIONEN
PROCÉDÉ D'OBTENTION DE NANO-ÉMULSIONS ULTRASTABLES

(30) Priority: 30.07.2012 IT TO20120673
(43) Date of publication of application: 10.06.2015
(73) Proprietor: Fondazione Istituto Italiano di Tecnologia, Genova (IT)
(72) Inventor: VECCHIONE, Raffaele, 16163 Genova (IT); NETTI, Paolo Antonio, 16163 Genova (IT); CIOTOLA, Ugo, 16163 Genova (IT); SAGLIANO, Angela, 16163 Genova (IT)
(74) Representative: Plebani, Rinaldo
(86) International application number: PCT/IB2013/056262
(87) International publication number: WO 2014/020543

(56) References cited:
- EP-A2- 1 120 102
- MCCLEMENTS D J ET AL: "Structured emulsion-based delivery systems: Controlling the digestion and release of lipophilic food components", ADVANCES IN COLLOID AND INTERFACE SCIENCE, ELSEVIER, NL, vol. 159, no. 2, 15 September 2010 (2010-09-15), pages 213-228, XP027232734, ISSN: 0001-8686 [retrieved on 2010-07-03]
- LEONG T S H ET AL: "Minimising oil droplet size using ultrasonic emulsification", ULTRASONICS: SONOCHEMISTRY, BUTTERWORTH-HEINEMANN, GB, vol. 16, no. 6, 1 August 2009 (2009-08-01) , pages 721-727, XP026139235, ISSN: 1350-4177, DOI: 10.1016/J.ULTSONCH.2009.02.008 [retrieved on 2009-02-27]
- JOHANNA GALINDO-ALVAREZ ET AL: "Enhanced stability of nanoemulsions using mixtures of non-ionic surfactant and amphiphilic polyelectrolyte", COLLOIDS AND SURFACES. A, PHYSICACHEMICAL AND ENGINEERING ASPECTS, ELSEVIER, AMSTERDAM, NL, vol. 389, no. 1, 18 August 2011 (2011-08-18), pages 237-245, XP028315358, ISSN: 0927-7757, DOI: 10.1016/J.COLSURFA.2011.08.021 [retrieved on 2011-08-25]

## Description

### Field of the Invention

The present invention concerns a method for obtaining, in a simple manner, nano-emulsions, typically of oil, fat and lipids in general, in water, which remain stable for periods in the order of one year and longer.

### Background of the Invention

The term "nano-emulsions" here and below indicates emulsions in which the drops of oil/fat which are dispersed in the aqueous phase have sub-micrometric dimensions below 200 nm and in which the resulting emulsion has a polydispersity index (dispersion index - below PDI) which, although it is not typically specified and depends on the product quality, is generally greater than 0.1.

Today, the development of nano-systems able to transport and release bioactive substances is arousing great interest and is becoming one of the most important objectives in many industries, mainly the food, cosmetics and pharmaceutical industries. For example, the ability to encapsulate materials (for example liposoluble materials) can be exploited in the food industry to produce food which is both healthy and tasty or, in the cosmetics industry, to improve the stability of various cosmetic ingredients (such as unsaturated fatty acids, vitamins) and improve the penetration of some of these ingredients, such as vitamins, increase the effectiveness and tolerability of UV filters on the surface of the skin and make the product aesthetically more attractive. Furthermore, it is known that the ideal dimensions of carriers for the administration of anti-cancer drugs are nanometric dimensions, in particular below 200 nm.

A further fundamental requirement for obtaining stable suspensions is monodispersity, essential for obtaining reliable accurate administration of drugs.

In short, nano-emulsions consisting of small lipid drops dispersed in an aqueous medium may already, per se, be suitable as bases for many types of food (for example milk, cream, beverages, condiments, flavourings), cosmetics (creams, milk, gel) and pharmaceutical products (injectable for both nutritional and therapeutic purposes and also for preventive purposes as adjuvants for vaccines, inhalable by nebulisation, applicable transdermally, ophthalmic, etc..), on condition that an acceptable stability of the emulsion is achieved.

After obtaining the emulsion, the lipid drops tend to recombine due to various causes, forming larger drops to the detriment of the smaller ones (a phenomenon known as Ostwald ripening) or larger aggregates (flocculation) or they may tend to collect on the surface of the emulsion (a phenomenon known as creaming). One of the most widely used methods to prevent destabilisation is the use of a surface-active agent or surfactant (emulsifier), which is adsorbed on the surface of the drops just formed, reducing the interfacial tension and forming around the same, if the surfactant is of the anionic or cationic type, an electrically charged protective layer which can help to prevent aggregation. A recent strategy to improve the stability of the emulsions is to coat the lipid drops with a polymeric layer which can be obtained by forming a "secondary" emulsion, i.e. remixing the "primary" emulsion previously obtained, adding to the same a polyelectrolyte having a polarity opposite to that of the surfactant previously used [David Julian McClements. Theoretical Analysis of Factors Affecting the Formation and Stability of Multilayered Colloidal Dispersions. Langmuir: the ACS journal of surfaces and colloids, 2005; Vol. 21, Issue 21; pp 9777-9785]. The right dosage of polyelectrolyte is considered of fundamental importance: in fact, if the polyelectrolyte is present in an insufficient amount, it does not completely coat the single lipid drops and/or does not coat all the drops, promoting even more easily phenomena of coalescence; analogously if the amount of polyelectrolyte is excessive, flocculation can occur which destabilizes the system. The "right" amount of polyelectrolyte is theoretically the one that produces complete coating of the surface of the oil drops without an excess of polymer in solution. Said concentration is identified, in the state of the art, in an empirical manner, measuring the so-called "Z potential" since it has been observed that this parameter increases, in absolute value, as the concentration of polyelectrolyte increases, and reaches a maximum at the concentration which, in theory, would allow coating of the entire surface of all the lipid drops present in the emulsion with a polymeric monolayer; said concentration is known as "saturation concentration". However, it has also been found that reaching the saturation concentration does not entail the achievement of an adequate and above all permanent over-time stability since, for kinetic reasons, before all the polymer is deposited, partially coated drops can coalesce. To remedy this problem, work is normally carried out in conditions of over-saturation of polyelectrolyte. The secondary emulsion obtained in this way has a low PDI but is nevertheless unstable for long periods because the polyelectrolyte is not uniformly distributed and deposited on the dispersed phase during mixing. The production of nanoemulsions having coated droplets is known from D.J. McClements et al. "Structured emulsion-based delivery systems: Controlling the digestion and release of lipophilic food components", Advances in Colloid and Interface Science 159, (2010), 213-228.

### Summary of the invention

The object of the invention is to overcome the limits of the known art, allowing over-time stable emulsions to be obtained in which the lipid drops have comparable nanometric dimensions and, therefore, a relatively low polydispersity index, preferably no higher than 0.1.
The invention therefore relates to a method for obtaining ultrastable nano-emulsions as specified in claim 1.

In particular the method according to the invention comprises the steps of:
i)- dispersing a first amount of a first fluid substance in a second amount, greater than the first amount, of a second fluid substance which is not miscible with the first fluid substance, in the presence of at least one ionic emulsifier preferably having chemical-physical affinities with the first fluid substance, so as to form a primary emulsion of drops of nanometric dimensions of the first fluid substance in a homogeneous phase defined by the second fluid substance and on the drops of which at least part of said at least one ionic emulsifier is adsorbed; and
ii)- mixing the primary emulsion thus formed with a solution of a polyelectrolyte in the second fluid substance, the polyelectrolyte being chosen so as to have a polarity opposite to that of the at least one ionic emulsifier, so as to form a secondary emulsion in which the drops of the first fluid substance are coated at least partly by a polymeric layer of polyelectrolyte.

According to the invention, furthermore, the method of the invention comprises the steps of:
iii)- leaving the previously obtained secondary emulsion to stand; and
iv)- processing the secondary emulsion so as to re-disperse the first fluid substance in the second fluid substance under the influence of a high shear stress.

Here and below, "high shear stress" means the mechanical stress which is normally applied to a fluid substance as it passes into a high-pressure homogenizer, operating at pressures just below 1000 bar.

Furthermore, according to a non-secondary aspect of the invention, and in combination with the above, step ii) is carried out in conditions of over-saturation of polymer/ polyelectrolyte, in other words the polyelectrolyte concentration by weight in the second fluid substance is chosen so as to be significantly greater than the theoretical concentration of polyelectrolyte necessary to saturate the primary emulsion (known technically as "saturation concentration"); said "saturation concentration" is previously experimentally identified according to a measurement of the "Z potential" performed in a known way on a plurality of samples of secondary emulsion prepared with increasing concentrations of polyelectrolyte. In particular, "significantly greater" means, here and below, a concentration at least double or triple the "saturation concentration" and, preferably, greater by one order of magnitude than the saturation concentration previously calculated according to a measurement of Z potential.

Lastly, the invention also relates to an ultrastable nano-emulsion which has been obtained according to the method of the invention and which comprises a first amount of a first fluid substance dispersed in a second amount, greater than the first amount, of a second fluid substance which is not miscible with the first fluid substance; the nano-emulsion furthermore containing at least one ionic emulsifier adsorbed on respective drops of the first fluid substance dispersed in the second fluid substance and at least one polyelectrolyte having polarity opposite to that of the ionic emulsifier and which forms a polymeric coating around the drops of the first fluid substance; the nano-emulsion of the invention having, in combination, the following measurable physical-chemical characteristics: a dimension of the drops of the first fluid substance coated by the ionic emulsifier and by the polyelectrolyte between 50 and 300 nm; a dimensional distribution of the drops such that the nano-emulsion has a polydispersity index below 0.1; and an over-time stability of the dimensional values of the drops of the first fluid substance longer than 1 year.

In particular, according to one aspect of the invention, nano-emulsions of the above-mentioned type can be obtained, in which the drops of the first fluid substance dispersed in the second fluid substance have at least three coating layers (and up to over ten layers) including, starting from the inside outward, a first layer which forms a polymeric coating around the drops on which the ionic emulsifier is adsorbed, consisting of a polyelectrolyte having polarity opposite that of the ionic emulsifier; at least one second layer consisting of a polysaccharide having polarity opposite that of the polyelectrolyte which forms the second layer; and at least one third layer consisting preferably of the same polyelectrolyte which forms the first layer.

### Brief description of the drawings

Further characteristics and advantages of the present invention will appear clear from the following description of non-limiting embodiments thereof provided purely by way of example and with reference to the figures of the accompanying drawings, in which:
- figure 1 illustrates by a block diagram the main steps of the method according to the invention; and
- figure 2 is a diagram which illustrates the properties of high over-time stability of the nano-emulsion according to the invention at different concentrations of a component.

### Detailed disclosure

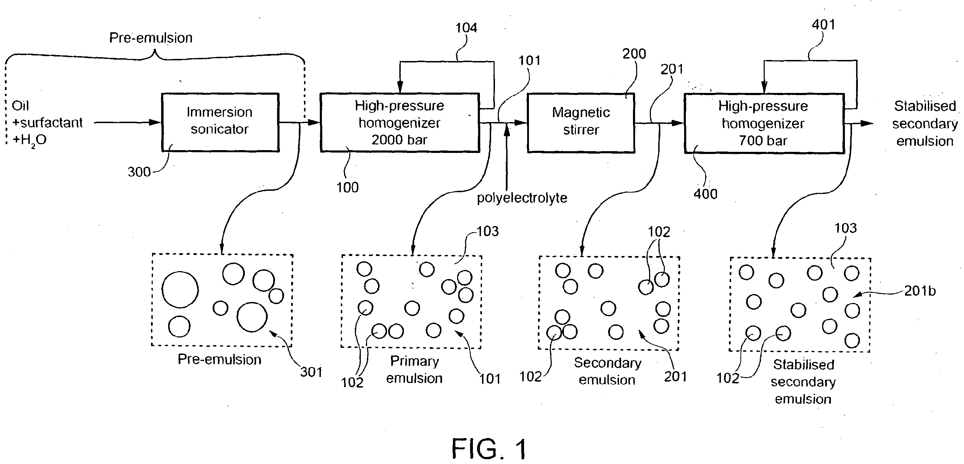

With reference to figure 1, the method according to the invention comprises a first step, represented by a block 100, in which a high-pressure homogenizer, operating for example at approximately 2000 bar, of substantially known type, performs the dispersion of a first amount of a first fluid substance, typically a lipid substance, in a second amount, greater than the first amount, of a second fluid substance which is not miscible with the first, for example water or an aqueous solution, in the presence of at least one ionic emulsifier of any known type preferably having chemical-physical affinity with the first fluid substance.

In the non-limiting example illustrated, the first fluid substance is an oil and the second fluid substance is water; in this way, at the outlet of block 100 a primary emulsion 101 is obtained (represented by the arrow and illustrated schematically and not to scale, below block 100) of drops 102 of nanometric dimensions of the first fluid substance in a homogeneous phase 103 defined by the second fluid substance and on the drops 102 of which at least part of the ionic emulsifier (surfactant) is adsorbed, not illustrated for the sake of simplicity. To achieve said result, the primary emulsion 101 is recycled several times, according to the arrow 104, in block 100, so that the first fluid substance is redispersed several times in the second fluid substance.

Subsequently, the primary emulsion 101 is mixed, in a second phase of the method, represented by a block 200, with a solution of a polyelectrolyte in the second fluid substance; the polyelectrolyte is chosen in order to have a polarity opposite to that of the ionic emulsifier, so that at the outlet of block 200 a secondary emulsion 201 forms in which the drops 102 of the first fluid substance are coated at least partly by a polymeric layer of polyelectrolyte, not illustrated for the sake of simplicity. Said phase is performed with a magnetic stirrer.

According to one aspect of the invention, the step performed in block 100 is preceded by a pre-emulsion step, illustrated by a block 300 in which the ionic emulsifier (surfactant) is previously dissolved in the first fluid substance and this solution is then slowly added to the second fluid substance. This mixture undergoes sonication; in the mixture (pre-emulsion 301) thus obtained there are considerable dimensional differences in the dispersed phase. The pre-emulsion 301 is passed through the high pressure homogenizer several times to obtain the primary emulsion 101.

The nano-emulsion 201 coming out of block 200 is not yet stable over time and could be subject to rapid deterioration of the constant dimensional characteristics of the drops 102, making it unusable.

According to the invention, therefore, in a subsequent block 400, the secondary nano-emulsion 201, after being left to stand for a pre-set period of time, is processed by means of a high-pressure homogenizer operating for example at approximately 700 bar, so as to re-disperse several times the first fluid substance in the second fluid substance under the influence of a high shear stress, performing in 400 a plurality of successive steps 401. At the end a "processed" secondary nano-emulsion 201b is obtained which, unlike the previous nano-emulsion 201, is now surprisingly stable over time since, as schematically shown in figure 1, the drops 102 of dispersed phase (oil) are uniformly distributed in the matrix 103 also in the long term. The secondary nano-emulsion 201b is therefore a stabilised secondary nano-emulsion.

It has been found that said over-time stability further improves if the phases of standing and re-dispersion with recycling in 400 and 401 are repeated, after the first execution, at least once and, preferably, at least twice.

According to a further characteristic of the invention, to obtain the surprising result described above, work must be carried out in conditions of over-concentration of the polyelectrolyte or other polymer used in preparation of the secondary nano-emulsion, i.e. the concentration by weight in polyelectrolyte in the second fluid substance at the time of mixing in 200 is chosen so as to be significantly greater than a theoretical concentration of polyelectrolyte known as "saturation concentration", corresponding to the concentration of polyelectrolyte necessary to theoretically saturate the primary emulsion, i.e. to coat all the drops 102.

Said "saturation concentration", which obviously depends on the chemical-physical nature of the first and second substance, of the polyelectrolyte and the ionic emulsifier is experimentally identified, previously, according to a method known to persons skilled in the art, on the basis of a measurement of Z potential performed on a plurality of samples of secondary emulsion 201 prepared with increasing concentrations of polyelectrolyte.

In particular, according to the method of the invention, firstly a plurality of samples of the secondary emulsion to be obtained are prepared, using increasing concentrations of polyelectrolyte; the theoretical concentration of polyelectrolyte necessary to saturate the primary emulsion (saturation concentration) is then experimentally identified by means of a known measurement of Z potential performed on said samples.

Subsequently, when preparing the secondary emulsion in 200, at this point a concentration of polyelectrolyte is used which is significantly greater than the saturation concentration previously determined (if not already known) by measurement of Z potential on the samples previously prepared.

Preferably, the concentration by weight in polyelectrolyte in the second fluid substance in the phase in 200 is chosen around an order of magnitude greater than the saturation concentration previously calculated according to a measurement of Z potential.

To obtain the nano-emulsions 101 and 201b, a high-pressure homogenizer is used operating at a first pressure which is chosen according to the chemical-physical nature of the first and second fluid substance and which is in any case in the range between 1000 and 3000 bar, preferably between 1500 and 2000 bar, to obtain the nano-emulsion 101; and operating at a second pressure, which is chosen according to the chemical-physical nature of the first and second fluid substance, but which is in any case lower than the first pressure, in the range between 200 and 1500 bar, preferably between 500 and 1200 bar and, even more preferably, between 700 and 1000 bar to obtain the nano-emulsion 201b; furthermore, the first and the second fluid substance are passed through the homogenizer several times in succession, so as to perform 3 to 15 complete homogenization cycles to obtain the nano-emulsion 101 and so as to perform at least 10 complete homogenization cycles to obtain the nano-emulsion 201b.

"Complete cycle" means passage through the homogenizer of the entire amount of substance in process. If working small quantities of substances, as in a laboratory, for example, to take account of the amount of emulsion that would remain trapped in the homogenizer, the recirculation operations in 104 and 401 are performed in continuous mode, feeding the flow at the outlet of the homogenizer directly to the reservoir at the homogenizer inlet. In this case, the 10-15 complete cycles mentioned above can correspond to 50-200 continuous recirculation passages or steps of the substance in process through the homogenizer, as can be easily calculated by a person skilled in the art who has access to the technical data of the homogenizer used and knows the quantities of substances in process. Obviously when the starting volume is changed, the number of steps necessary must be re-identified, verifying the number of steps that minimise dimensions and PDI. The complete cycles, on the other hand, remain independent of the volume as the liquid passes entirely without remixing the outflow with the substance which has still not been processed and is in the reservoir. Combinations of the two types of cycle - complete cycles and continuous cycles - can be used if necessary.

The standing phase between blocks 200 and 400 can cover a time range of between a few hours and 1 month.

In practice, the nano-emulsion 201 is left to stand for 1 day before carrying out the processing in 400; the subsequent re-processing operations after the first one are performed after standing phases which can last from one week to one month.

According to the preferred embodiment of the invention, the first fluid substance is a lipid substance (for example soy oil) and the second fluid substance is an aqueous substance (for example distilled water or an aqueous solution) ; in this case the ionic emulsifier is chosen from the group consisting of: soy lecithin, lecithin enriched with phosphatidylcholine (PC) and/or phosphatidylethanolamine (PE) and mixtures thereof; if necessary, glycerol, pectin or other thickening agents can be added to the second fluid substance. Furthermore, the polyelectrolyte is chosen from the biodegradable polyelectrolytes, such as polysaccharides and proteins. Some examples are chitosan, polylysine, heparin and alginates, for example sodium alginate.

In short, it has been found that, with the above substances, to obtain stable nano-emulsions of good quality the following parameters of the method described above must be taken into account:
- Pressure of the homogenizer in 400: the optimal pressure depends on the interaction between the polymer and the surface of the emulsion (on which, in this case, lecithin is present). The stronger the interaction, the higher the pressure that can be reached without the risk of the polymer detaching from the surface, producing a deterioration rather than an improvement in the monodispersion. In our case the optimal pressure is between 700 and 1000 bar. Lower pressures are not able to guarantee effective disaggregation of the weak aggregates. Higher pressures lead to deterioration. For other formulations with different interactions it may be possible to extend the working pressure range even to between 200 and 1500 bar.

- Number of re-dispersion cycles (in 401): also in this case it is possible to identify an optimal number of cycles. For example it has been experimentally observed in the laboratory that a number of cycles in continuous mode or steps equal to 100 may be optimal, given that a lower number has produced the desired benefits, while a higher number could produce an effect similar to that of excessive pressures or deterioration in the dispersion. In general, it can be affirmed that a suitable number of cycles in continuous mode or steps is between 50 and 200 and, more preferably, between 75 and 150, but wider optimal ranges are possible in the case of different formulations. In short, the number of cycles depends on the type of cycle and type of machine (for example the volume of the reservoir). It is therefore determined by verifying the number of cycles that gives the minimum PDI. Furthermore, it is possible, as already indicated, to combine complete and "continuous" cycles, as previously defined.
- Number of re-dispersions and time between the re-dispersions: it has been seen that with a double re-dispersion process, after a few days/weeks it was possible to further improve the stability of the product. The number of repetitions of the standing and re-dispersion phases can therefore be considered between 1 and 5, with time between one re-dispersion and another ranging from a few hours to 1 month. A time interval of a few hours is not always optimal since the kinetics are probably not able to guarantee a configuration of partial stability on which to operate. An excessive period of time (>1 month) could result in stable aggregates which cannot be recovered, even by re-processing.

With the method described, thanks to the reprocessing, a stabilised secondary emulsion is obtained which becomes an optimal base for performing in an analogous manner a deposition of polyelectrolyte multilayer with opposite charge, for example for specific technological sectors such as the pharmaceutical sector. In this case, the drops of the first fluid substance dispersed in the second fluid substance have at the end, in addition to the layer of surfactant, at least three coating layers including, from the inside outward, a first layer that forms a polymeric coating and consisting of the polyelectrolyte having opposite polarity to that of the ionic emulsifier; at least one second layer consisting of a polysaccharide having polarity opposite to that of the polyelectrolyte which forms the second layer; and at least one third layer consisting of a polysaccharide having polarity opposite to that of the polyelectrolyte which forms the second layer.

Nano-emulsions have been obtained preliminarily, for example, in which the first polymeric layer is formed of chitosan, the second polymeric layer is formed of sodium alginate or heparin and in which a third layer is formed of chitosan.

The invention will now be further described by means of a series of practical implementation examples.

### EXAMPLES

A series of O/W (oil in water) emulsions are prepared using soy oil (density at 20°C of 0.922 g/ml) and surface-active agent Lipoid E80 (egg lecithin powder 80-85% by weight enriched with phosphatidylcholine (PC) and 7-9.5% by weight of content in phosphatidylethanolamine (PE) as indicated in the manufacturing specifications) purchased from Lipoid and used without further purification. Distilled water Millipore® "Milli-Q" was used for preparation of all the emulsions and solutions. As polyelectrolytes, Chitosan (CT, LMW 90-150 kDa, DDA 84% determined by means of 1H-NMR) was used, which is a casual linear copolymer of D-glucosamine and N-acetyl-D-glucosamine (pKa approximately 6.7).

Firstly an oily phase was prepared with the addition of a certain amount of Lipoid E 80, previously weighed with a precision balance, to a volume of soy oil. The oily phase was mixed (for 10 minutes at a temperature of 60°C and at a speed of 500 rpm) using a high speed mixer (RZR 2102 control, Heidolph) and then, after reaching ambient temperature, sonicated for 1.5 min (sonication amplitude 60%, and mixed with the impulse sonicator in on-off for 10 s) with an immersion sonicator (ultrasound processor VCX 500 or 750). To obtain the pre-emulsion, the oily phase was added progressively to a weighed amount of aqueous phase (Milli-Q water) under sonication during the first 3 minutes (sonication amplitude 70%; on-impulse and off-impulse 10 and 5 s respectively) and then for a further 5 minutes under the same conditions. To avoid over-heating of the pre-emulsion during sonication, the preparation was performed in a large beaker around which an ice jacket was created.

The same procedure was performed for verification with alternative techniques (Turrax, Silverson), which allow larger volumes to be handled than with the immersion sonicator, obtaining similar results.

Lastly, the pre-emulsion was passed at 2000 bar through a high pressure valve homogenizer (Microfluidics M110PS) for a first three single cycles to greatly reduce the initial dimension and then the operation was repeated, re-filling the reservoir in continuous mode for several steps, thus recycling the product without any loss of volume, recovering small volumes of emulsion after 50, 100, 150 and 200 recirculations or successive steps. These volumes were sampled to monitor the best process condition and at the end the best result was the one with 200 steps. This number is arbitrary since it depends on the volume re-processed. The guiding criterion is therefore that of verifying the minimum dimension and minimum PDI reached with sampling of the volumes, identifying once and for all the optimal number of steps.

This method was used for preparation of all the oil-in-water emulsions at different oily phase concentrations (10% by weight, 20% by weight, 30% by weight) . The emulsions with oil at different lecithin concentration are classified according to the "Lipoid E80/Oily Phase" ratio which remains constant at all the oil concentrations, as reported in Table 1.

**TABLE 1**

| **Emulsion name** | **Lipoid E80 /Oily Phase (g/ml)** |
|---|---|
| L1 | 0.06 |
| L2 | 0.12 |
| L3 | 0.18 |
| L4 | 0.24 |
| L5 | 0.3 |

A 1% by weight solution of chitosan (CT) in a solution of acetic acid and water at pH 2.5 (0.5 M acetic acid) is prepared dissolving 500 mg of purified chitosan dried in 50 ml of aqueous solution of acetic acid (0.10 M).

Secondary emulsion: to evaluate the optimal concentration of chitosan to be deposited, according to the "saturation method", 0.5M solutions of acetic acid at different concentrations of chitosan (0.0025%, 0.005%, 0.00.875 thousand%, 0.0125%, 0.025%, 0.05%, 0.0875% , 0.125% 0.250%, 0.5%, 0.875%, 1.25% by weight) were prepared and brought to pH 4 with NaOH 24M. Volumes of 9 ml of these solutions were diluted 1:4 with a solution at pH 4 of acetic acid, bringing them to 36 ml. 9 ml of primary emulsion diluted to 5% in oily phase were then added rapidly to the 36 ml of solution under vigorous stirring, stirring everything for 15 minutes to allow uniform deposition of chitosan. The final secondary emulsions have a pH of 4. The emulsions thus obtained were passed through the high pressure valve homogenizer used previously, but at a pressure of 700 bar for 100 cycles or recirculation steps in continuous mode, and then re-treated after one month under the same conditions.

The Z potential of the nano-capsules (coated drops) forming the emulsion was determined using a particle electrophoresis instrument (ZetaDim.r zs nano series ZEN 3600, Malvern Instruments Ltd., Malvern, United Kingdom). The Z potential is determined by measuring the direction and the speed of movement of the drops in a well-defined electrical field. The Z potentials measured are reported as a mean and standard deviation of 3 measurements of 50 runs each. Before the measurements, the emulsions are diluted at a concentration in drops of approximately 0.025% by weight using Milli-Q water and solutions of acetic acid in Milli-Q (ph 4, 6.5x10-4M). The dimensional distribution of the emulsions and relative nano-capsules was measured using a laser instrument with dynamic light scattering (DLS) (λ = 632.8 nm). This instrument correlates the fluctuations of intensity I(t) of light dispersed over time with the Brownian motion of the colloidal particles and then with the coefficient of diffusion D linked to the hydrodynamic radius Rh of the particles. To avoid diffusion effects, a detecting angle of 173° was used. A refractive index with pre-defined ratio (1,52) was used in calculation of the granulometric distribution. For each measurement the dimension of the particles is reported as a mean dimension and the index of polydispersity (PDI) is on 3 measurements each on 5 lanes (1 run per lane for 100 s).

The different concentrations indicated were tested on emulsions of five different dimensions, which were re-processed the first time after one day of standing; part of this volume was then re-processed for a second time after one month.

The results obtained were reported in tables 2 and 3 below.

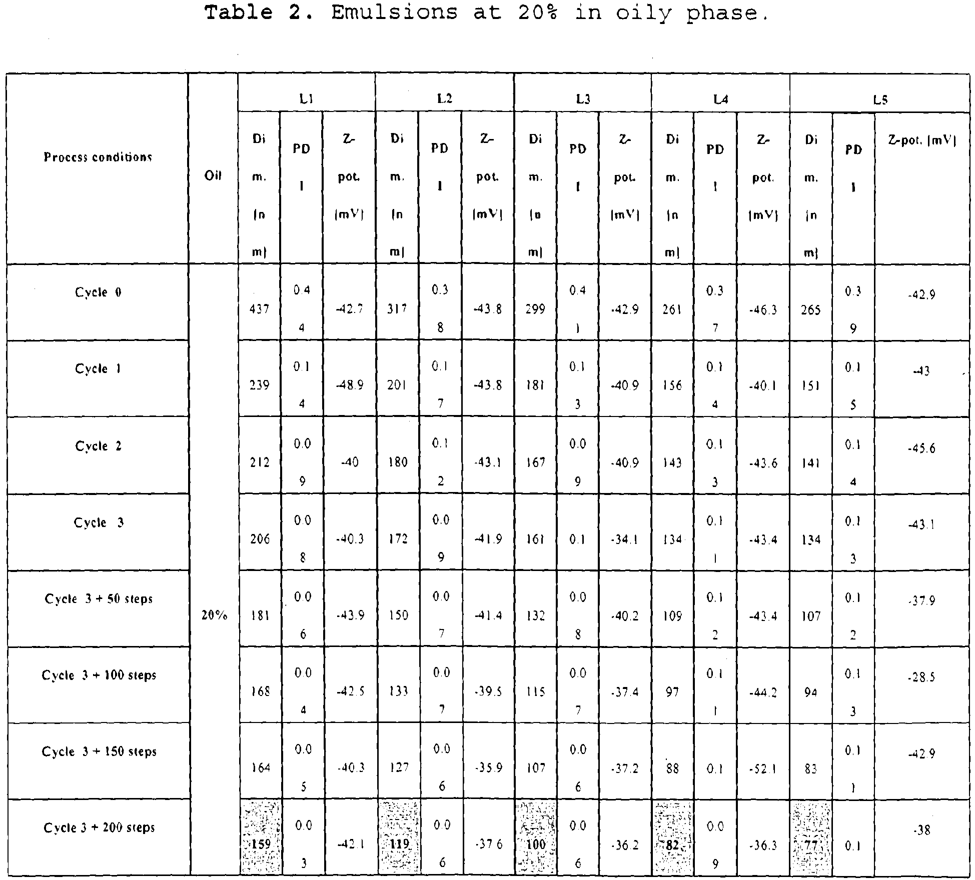

**Table 3**

| L1 coated in chitosan passed once through the homogenized (A) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **TIME 0** | 188.4 | 0.07 | 172.4 | 006 | 180.7 | 0 05 | 174.2 | 0.04 | 173.2 | 0.05 | 182.9 | 0.1 | 199.3 | 0.11 | 197.6 | 0.11 |
| **1 day** | 215.9 | 0.12 | 195.6 | 008 | 192.7 | 0.08 | 185.8 | 0.09 | 186.7 | 0.10 | 191.2 | 0.13 | 220.4 | 0.12 | 249.1 | 0.16 |
| **3 days** | 199.2 | 0.09 | 193.1 | 0.1 | 210.8 | 0,20 | 190.8 | 0.08 | 191.1 | 0.09 | 200 | 0.1 | 237.2 | 0.13 | 250 | 0.14 |
| **7 days** | 208.6 | 0.08 | 197.1 | 0.09 | 200.2 | 0.08 | 189.4 | 0.07 | 191.3 | 0.09 | 195.2 | 0.12 | | | 249.2 | 0.17 |
| **14 days** | 209.5 | 0.11 | 205.6 | 008 | 207.1 | 0.1 | 203.8 | 0.09 | 196.7 | 0.1 | 202.5 | 0.15 | | | 242.2 | 0.11 |
| **28 days (t0)** | 215.1 | 0.10 | 204.1 | 0.06 | 206.8 | 0.07 | 204.6 | 0.07 | 202.1 | 0.09 | 195.5 | 0.10 | | | 247.2 | 0.12 |
| **75 days (t1)** | | | | | 638.8 | 0.41 | 198.6 | 0.08 | 211 | 0.08 | 214.8 | 0.08 | | | 254.2 | 0.08 |
| **90 days (t2)** | | | | | | | 338.7 | 0.35 | 205.5 | 0.04 | 224.8 | 0.06 | | | 248.3 | 0.07 |
| **160 days (t3)** | | | | | | | | | 208.9 | 0.12 | 214 | 0.09 | | | 229 | 0.12 |
| **190 days (t4)** | | | | | | | | | 286.4 | 0.32 | 197.1 | 0.1 | | | 201.7 | 0.1 |
| **270 days (t5)** | | | | | | | | | | | 211.6 | 0.04 | | | 219.3 | 0.06 |
| **1 year (t6)** | | | | | | | | | | | 214.3 | 0.1 | | | 214.2 | 0.07 |

| L1 coated in chitosan re-processed in the homogenizer after 1 month (B) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **[28] days (t0)** | 214.4 | 0.1 | 195.6 | 0.08 | 183.9 | 0.06 | 187 | 0.06 | 185.7 | 0.08 | 186.5 | 0.07 | | | 206.4 | 0.07 |
| **31 days** | 247.4 | 0.11 | 1894 | 0.09 | 194.1 | 0.08 | 190.1 | 0.08 | 181.9 | 0.07 | 184.4 | 0.07 | | | 200. 7 | 0.06 |
| **42 days** | | | 193.7 | 0.09 | 191.2 | 0.07 | 190.8 | 0.07 | 193 | 0.15 | 178.8 | 008 | 181.4 | 0.08 | 206 | 0.12 |
| **60 days (t1)** | | | | | 189.7 | 0.06 | 183 | 0.1 | 184.5 | 0.07 | 182.3 | 0.08 | 181.9 | 0.07 | 196.7 | 0.08 |
| **95 days (t2)** | | | | | | | 182.2 | 0.08 | 181.2 | 009 | 177.7 | 0.08 | 170.5 | 0.08 | 192.5 | 0.12 |
| **161 days (t3)** | | | | | | | | | 175 | 0.11 | 174 | 009 | 167.8 | 0.09 | 177.5 | 0.09 |
| **190 days (t4)** | | | | | | | | | 178.2 | 0.07 | 174.4 | 0.12 | 168 | 0.07 | 181.5 | 0.12 |
| **270 days (t5)** | | | | | | | | | | | **180.2** | 0.08 | 170.8 | 0.08 | 185.9 | 0.09 |
| **1 year (t6)** | | | | | | | | | | | **178.7** | **0.12** | **165** | **0.06** | **174..2** | **0.07** |

| L2 coated in chitosan passed once through the homogenizer (C) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **TIME 0** | 134.8 | 0.11 | 128.4 | 0.08 | 128.7 | 0.06 | 127 | 0.06 | 125.1 | 0.1 | 139.5 | 0.12 | 162 | 0.17 | 196.8 | 0.17 |
| **1 day** | 134.4 | 0.10 | 130.3 | 0.09 | 131.3 | 0.06 | 132.2 | 0.07 | 128.7 | 0.09 | 138.3 | 0.11 | 167.6 | 0.16 | 189.1 | 0.17 |
| **3 days** | 143.3 | 0.07 | 138.1 | 0.06 | 139.1 | 0.09 | 139.1 | 0.06 | 136.8 | 0.09 | 148.8 | 0.12 | 179.7 | 0.16 | 206.6 | 0.16 |
| **7 days** | 148.2 | 0.11 | 135.7 | 0.09 | 131.9 | 0.07 | 141.7 | 0.08 | 143 | 0.09 | 155.1 | 0.11 | 185.4 | 0.14 | 203.8 | 0.15 |
| **14 days** | 165.6 | 0.07 | 139.3 | 0.08 | 149.8 | 0.12 | 142.7 | 0.07 | 142.8 | 0.08 | 148.7 | 0.13 | 172.9 | 0.14 | 198 | 0.16 |
| **28 days (t0)** | | | 134.9 | 005 | 1374 | 0.07 | 145.5 | 0.07 | 150.1 | 0.09 | 154 7 | 0.08 | 171.9 | 0.12 | 193.6 | 0.15 |
| **75 days (t1)** | | | | | | | 190.1 | 0.1 | 148.8 | 0.08 | 165.8 | 0.07 | 179.5 | 0.07 | 194.3 | 0.13 |
| **90 days (t2)** | | | | | | | | | 144.7 | 0.06 | 166.8 | 0.06 | 177 | 0.09 | 187.7 | 0.13 |
| **157 days (t3)** | | | | | | | | | 262.3 | 0.36 | 163.3 | 0.12 | 160.5 | 0.07 | 197.9 | 0.22 |
| **190 days (t4)** | | | | | | | | | | | 142.4 | 0.13 | 146 | 0.09 | 151.6 | 0.11 |
| **270 days (t5)** | | | | | | | | | | | 159.5 | 0.08 | 163.7 | 0.07 | 165.2 | 0.09 |
| **1 year (t6)** | | | | | | | | | | | 160.4 | 0.1 | 152.9 | 0.07 | 162.8 | 0.17 |

| L2 coated in chitosan re-processed in the homogenizer after 1 month (D) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **28 days (t0)** | | | 134.1 | 0.06 | 134.4 | 0.08 | 132.3 | 0.03 | 127 | 0.07 | 128.3 | 0.08 | 145.3 | 0.09 | 156.7 | 0.09 |
| **31 days** | | | 134.1 | 0.07 | 135 | 0.06 | 136.1 | 0.04 | 135.7 | 0.07 | 134.6 | 0.08 | 147.6 | 0.09 | 158.2 | 0.09 |
| **42 days** | | | | | 132 | 0.06 | 130.6 | 0.05 | 131.2 | 0.08 | 135.5 | 0.11 | 141.5 | 0.09 | 153.4 | 0.09 |
| **57 days (t1)** | | | | | | | 128.9 | 0.06 | 127.9 | 0.04 | 131 | 0.06 | 137.6 | 0.09 | 145.8 | 0.09 |
| **92 days (t2)** | | | | | | | | | 129.9 | 0.11 | 131.3 | 0.12 | 136.5 | 0.14 | 142.9 | 0.14 |
| **158 days (t3)** | | | | | | | | | 121.6 | 0.07 | 122.5 | 0.04 | 124.8 | 004 | 133.3 | 0.15 |
| **190 days (t4)** | | | | | | | | | 128.7 | 0.06 | 125 | 0.09 | 125.1 | 0.06 | 136.4 | 0.16 |
| **270 days (t5)** | | | | | | | | | | | 127.8 | 0.06 | 117.8 | 0.17 | 137.7 | 0.14 |
| **1 year (t6)** | | | | | | | | | | | **129.7** | **0.09** | **126.3** | **0.12** | 131.2 | 0.14 |

| L3 coated in chitosan passed once through the homogenizer (E) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **TIME 0** | 102.5 | 0.08 | 101.9 | 0.08 | 103.4 | 0.1 | 102.6 | 0.10 | 108.7 | 0.12 | 115.4 | 0.13 | 126.4 | 0.18 | 142 | 0.2 |
| **1 day** | 103.2 | 0.1 | 105.4 | 0.09 | 106.1 | 0.09 | 101.6 | 0.08 | 111.4 | 0.12 | 122 | 0.15 | 134.1 | 0.18 | 156 | 0.19 |
| **3 days** | 108.6 | 0.09 | 107 | 0.09 | 110.2 | 0.08 | 111.2 | 0.13 | 110.2 | 0.11 | 125.4 | 0.14 | 144 | 0.17 | 167.3 | 0.12 |
| **7 days** | 117.4 | 0.06 | 107.9 | 0.08 | 114 | 0.09 | 111.7 | 0.01 | 111.7 | 0.11 | 123.9 | 0.15 | 147.1 | 0.17 | 170.2 | 0.17 |
| **14 days** | 226.3 | 0.32 | 111.2 | 0.07 | 111.5 | 0.06 | 109.5 | 0.07 | 106.4 | 0.08 | 123.2 | 0.13 | 128.4 | 0.15 | 127.9 | 0.14 |
| **28 days (t0)** | | | 126.3 | 0.07 | 115.8 | 0.08 | 120.6 | 0.06 | 125 | 0.08 | 132 | 0.12 | 149.4 | 0.14 | 169.5 | 0.15 |

| L3 coated in chitosan re-processed in the homogenizer after 1 month (F) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **28 days (t0)** | | | 139 | 0.1 | 111 | 0.07 | 109.9 | 0.07 | 111.3 | 0.08 | 113.6 | 0.1 | 121 | 0.11 | 131.7 | 0.12 |
| **31 days** | | | 218.9 | 0.12 | 108.5 | 0.1 | 106.5 | 0.07 | 108.2 | 0.07 | 110.8 | 0.09 | 121 | 0.13 | 131.5 | 0.1 |
| **42 days** | | | | | 280.7 | 0.17 | 104.2 | 0.07 | 109.2 | 0.07 | 109.3 | 0.09 | 114.1 | 0.11 | 124.2 | 0.1 |
| **56 days (t1)** | | | | | | | 155.3 | 0.05 | 107.8 | 0.08 | 107.4 | 0.1 | 111.7 | 0.11 | 115.2 | 0.1 |
| **85 days (t2)** | | | | | | | | | 106 | 0.1 | 105.9 | 0.09 | 110.3 | 0.11 | 123.3 | 0.18 |
| **152 days (t3)** | | | | | | | | | 101.7 | 0.07 | 99.09 | 0.06 | 103.9 | 0.01 | 104.6 | 0.08 |
| **190 days (t4)** | | | | | | | | | 113.9 | 0.07 | 100.2 | 0.074 | 102.1 | 0.07 | 106.3 | 0.1 |
| **270 days (t5)** | | | | | | | | | | | **105.3** | 0.09 | **102** | 0.07 | **101.8** | 0.06 |
| **1 year (t6)** | | | | | | | | | | | 115.7 | 0.14 | **106.2** | **0.09** | **100.7** | **0.07** |

| L4 coated in chitosan passed once through the homogenizer (G) | | | | | | | | | | | | | | | | |
|---|---|---|---|---|---|---|---|---|---|---|---|---|---|---|---|---|
| **Chitosan concentration[%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **TIME 0** | 87.81 | 0.01 | 88.28 | 0.12 | 89.78 | 0.13 | 89.28 | 0.11 | 91.57 | 0.15 | 95.81 | 0.15 | 104.8 | 0.19 | 110.3 | 0.2 |
| **1 day** | 89.78 | 0.09 | 91.08 | 0.11 | 91.96 | 0.11 | 91.8 | 0.12 | 94.73 | 0.14 | 107.4 | 0.2 | 111.9 | 0.19 | 131.2 | 0.22 |
| **3 days** | 106.3 | 0.09 | 94.03 | 0.10 | 95.66 | 0.11 | 96.23 | 0.11 | 103 | 0.17 | 110 | 0.17 | 131.9 | 0.1 | 149.6 | 0.19 |
| **7 days** | 172.8 | 0.13 | 104.5 | 0.09 | 101.4 | 0.1 | 93.59 | 0.11 | 104.7 | 0.17 | 119.1 | 0.21 | 146.3 | 0.23 | 193.8 | 0.35 |
| **14 days** | | | 113 1 | 0.08 | 100.1 | 0.11 | 101.9 | 0.1 | 108.9 | 0.15 | 111.4 | 0.16 | 132.3 | 0.17 | 151.9 | 0.18 |
| **28 days (t0)** | | | | | 102.3 | 0.08 | 103.2 | 0.09 | 110.9 | 0.11 | 117.3 | 0.13 | 131.5 | 0.16 | 142.9 | 0.16 |
| **68 days (t1)** | | | | | | | | | 110.8 | 0.15 | 115 | 0.09 | 135.6 | 0.14 | 144.9 | 0.11 |
| **88 days (t2)** | | | | | | | | | 167.4 | 0.13 | 117.2 | 0.11 | 126.1 | 0.1 | 138.5 | 0.1 |
| **150 days (3)** | | | | | | | | | | | 119.3 | 0.07 | 118.7 | 0.08 | 122 | 0.08 |
| **190 days (t4)** | | | | | | | | | | | 137.6 | 0.09 | 123.5 | 0.08 | 127.7 | 0.06 |
| **270 days (t5)** | | | | | | | | | | | 219.6 | 0.26 | 128.6 | 0.06 | 127.4 | 0.06 |
| **1 year (t6)** | | | | | | | | | | | | | 134.5 | 0.1 | 122.4 | 0.09 |

| L4 coated in chitosan re-processed in the homogenizer after 1 month (H) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **28 days (t0)** | | | 252.9 | 0.05 | 97.48 | 0.1 | 95.56 | 0.08 | 96.8 | 0.1 | 98.13 | 0.11 | 105.3 | 0.13 | 113.9 | 0.13 |
| **31 days** | | | | | 95.51 | 0.07 | 94.37 | 0.08 | 96.51 | 0.08 | 96.83 | 0.12 | 104 | 0.14 | 114 | 0.14 |
| **42 days** | | | | | | | 91.58 | 0.07 | 90.49 | 0.09 | 92.92 | 0.1 | 97.72 | 0.11 | 106.8 | 0.12 |
| **56 dans (t1)** | | | | | | | | | 91.19 | 0.11 | 91.34 | 0.11 | 99.12 | 0.13 | 104.6 | 0.14 |
| **84 days (t2)** | | | | | | | | | 90.26 | 0.14 | 91.3 | 0.12 | 85.68 | 0.12 | 105.4 | 0.19 |
| **151 days (t3)** | | | | | | | | | 101.4 | 0.06 | 84.88 | 0.08 | 87.62 | 0.01 | 91.19 | 0.01 |
| **190 days (t4)** | | | | | | | | | 175.5 | 0.1 | 89.93 | 0.15 | 88.32 | 0.11 | 92.6 | 0.13 |
| **270 days (t5)** | | | | | | | | | | | 91.73 | 0.12 | 88.98 | 0.1 | 89.45 | 0.1 |
| **1 year (t6)** | | | | | | | | | | | 98.59 | 0.15 | **92.53** | **0.1** | **91.08** | **0.1** |

| 5 coated in chitosan passed once through the homogenizer (I) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **TIME 0** | 84.58 | 0.1 | 83.19 | 0.12 | 83.61 | 0 13 | 82.27 | 0.12 | 81.87 | 0.12 | 88.42 | 0.17 | 95.38 | 0.18 | 108.2 | 0.21 |
| **1 day** | 85.38 | 0.12 | 84.79 | 0.11 | 83.17 | 0.11 | 85.01 | 0.11 | 86.27 | 0.13 | 96.97 | 0.19 | 107.4 | 0.22 | 122.8 | 0.21 |
| **3 days** | 1034 | 0.18 | 91.28 | 0.14 | 90.15 | 0.14 | 84.76 | 0.11 | 93.41 | 0.15 | 101 | 0.19 | 117.8 | 0.21 | 147 | 0.22 |
| **7 days** | 160.5 | 0.14 | 98.6 | 0.1 | 89. 19 | 0.1 | 92.52 | 0.1 | 92.85 | 0.13 | 92.18 | 0.15 | 112.5 | 0.2 | 127.9 | 0.2 |
| **14 days** | | | 107.7 | 0.07 | 96.07 | 0.11 | 97.67 | 0.1 | 99.6 | 0.13 | 106.6 | 0.15 | 137.1 | 0.23 | 133.9 | 0.17 |
| **28 days (t0)** | | | | | 101.3 | 0.07 | 97.54 | 0.1 | 97.19 | 0.09 | 100.9 | 0.13 | 104.2 | 0.14 | 131.6 | 0.16 |
| **72 days (t1)** | | | | | | | | | 142.4 | 0.09 | 116 | 0.13 | 125.8 | 0.12 | 132.2 | 0.12 |
| **87 days (t2)** | | | | | | | | | | | 114.2 | 0.14 | 126.1 | 0.15 | 129 | 0.11 |
| **146 days (t3)** | | | | | | | | | | | 138.9 | 0.14 | 115.3 | 0.09 | 117.5 | 0.1 |
| **190 days (t4)** | | | | | | | | | | | 219.7 | 0.25 | 124.7 | 0.1 | 124.4 | 0.15 |
| **270 days (t5)** | | | | | | | | | | | | | 133.3 | 0.08 | 123.5 | 0.06 |
| **1 year (t6)** | | | | | | | | | | | | | 155.4 | 0.1 | | |

| L5 coated in chitosan re-processed in the homogenizer after 1 month (L) | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **28 days (t0)** | | | | | 96.98 | 0.1 | 90.44 | 0.09 | 88.33 | 0.11 | 92.05 | 0.13 | 98.16 | 0.13 | 107.6 | 0.15 |
| **31 days** | | | | | 98.5 | 0.12 | 92.05 | 0.13 | 91.28 | 0.09 | 92.97 | 0.13 | 98.49 | 0.13 | 112.7 | 0.16 |
| **42 days** | | | | | | | 125 | 0.06 | 87.07 | 0.08 | 85.53 | 0.1 | 93.93 | 0.11 | 101.1 | 0.14 |
| **56 days (t1)** | | | | | | | | | 86.53 | 0.08 | 88.08 | 0.09 | 90.94 | 0.11 | 101.8 | 0.13 |
| **84 days (t2)** | | | | | | | | | 82.65 | 0.11 | 83.52 | 0.12 | 89.13 | 0.13 | 94.01 | 0.11 |
| **146 days (t3)** | | | | | | | | | | | 79.13 | 0.09 | 81.07 | 0.07 | 84.59 | 0.09 |
| **190 days (t4)** | | | | | | | | | | | 82.13 | 0.11 | 89.04 | 0.19 | 84.88 | 0.09 |
| **270 days (t5)** | | | | | | | | | | | 92.59 | 0.15 | 88.05 | 0.12 | 85.89 | 0.09 |
| **1 year (t6)** | | | | | | | | | | | 104.7 | 0.19 | 96.62 | 0.18 | **91.64** | **0.12** |

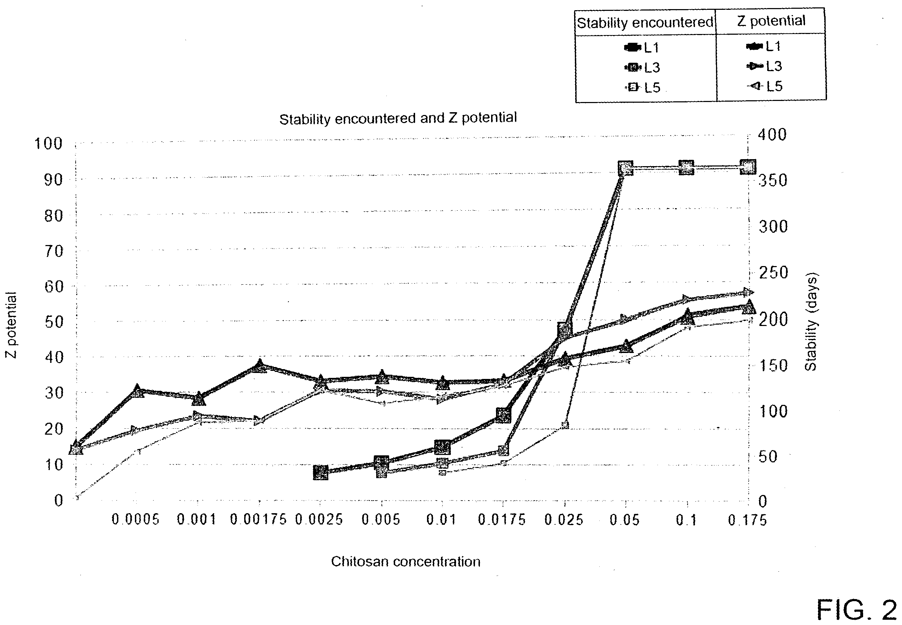

As can be observed, thanks to the single re-processing and in particular to the double re-processing, nano-emulsions which are surprisingly stable in the long term, for over 1 year, are obtained. Figure 2 shows the stability trend (number of days the nano-emulsion conditions were maintained) according to the % of chitosan used compared with the trend of the Z potential of the same emulsions, highlighting how maximum stability is reached well beyond the saturation concentration indicated by the trend of the Z potentials.

With the method described, thanks to the re-processing, an excellent base (the stabilised secondary emulsion) can be obtained for subsequent use for performing multilayer depositions of polyelectrolyte with opposite charge, for example for specific technological sectors such as the pharmaceutical sector. Multi-layer secondary emulsions are therefore obtained in which the drops of substance in dispersion have from three to five, six and even ten superimposed layers of coating including compounds chosen from the group consisting in chitosan, sodium alginate, heparin, polylysine and other polymers of comparable molecular weight and biodegradable.

To demonstrate the effectiveness of re-processing of the secondary emulsions, stability tests were also carried out, performed as an example on only two emulsions, i.e. those corresponding to L1 and L3.

The same concentrations as those reported in Table 3 were directly tested, once and twice. The data, for both L1 and L3, are reported in Table 4. At each concentration of polymer, the volume was divided into two parts and one of these two parts was processed the day after by simple sonication in an ultrasound bath (as reported at times in literature).

The results reported in Table 4 indicate the presence of a double dimensional peak (index of aggregations) while the data in italics indicate that, in addition to the second dimensional peak, the sample visually presents evident segregations, although it is still measurable as a dispersion.

**Table 4**

| L1 | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **Time 0** | 192 | 0.22 | 182.7 | 0.08 | 184.5 | 0.11 | 179.3 | 0.08 | 187.7 | 0.12 | 190.9 | 0.13 | 203 | 0.15 | 236.1 | 0.22 |
| **1 day** | *210.9* | *0.26* | 188.6 | 0.12 | 197.2 | 0.11 | 184.4 | 0.1 | 181.3 | 0.11 | 187.3 | 0.12 | 253.9 | 0.2 | 296.5 | 0.4 |
| **3 days** | | | 178.4 | 0.05 | 183.2 | 0.09 | 176.2 | 0.07 | 185.5 | 0.1 | 188.2 | 0.1 | 232.6 | 0.19 | 324.9 | 0.29 |
| **7 days** | | | 197.5 | 0.13 | 191.1 | 0.15 | 186.2 | 0.11 | 203.8 | 0.11 | 203.3 | 0.11 | 254.2 | 0.23 | 324.8 | 0.4 |
| **14 days** | | | | | | | | | | | | | | | | |
| **30 days** | | | | | | | | | | | | | | | | |

| L1 after sonication at 75% | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **Time 0** | 402.4 | 0.4 | 187.4 | 0.12 | 190.8 | 0.15 | 174.9 | 0.07 | 202 | 0.15 | 197.8 | 0.14 | 242.1 | 0.2 | 299.5 | 0.36 |
| **1 day** | | | 294.6 | 0.36 | 180.6 | 0.11 | 183.5 | 0.07 | 187 | 0.09 | 203.2 | 0.14 | 252.4 | 0.18 | 348.8 | 0.28 |
| **3 days** | | | 181.2 | 0.07 | 179.2 | 0.07 | 178.6 | 0.06 | 185.3 | 0.1 | 187 | 0.09 | 236.5 | 0.2 | 372.3 | 0.3 |
| **7 days** | | | 179.8 | 0.06 | 184.8 | 0.12 | 176.1 | 0.04 | 188.6 | 0.11 | 216.3 | 0.13 | 256.4 | 0.19 | 325.5 | 0.27 |
| **14 days** | | | | | | | | | | | | | | | | |
| **30 days** | | | | | | | | | | | | | | | | |

| L3 | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **Time 0** | 107.2 | 0.2 | 118.4 | 0.2 | 97.8 | 0.15 | 99.3 | 0.17 | 96.7 | 0.17 | 100.3 | 0.19 | 116.5 | 0.18 | 130.2 | 0.25 |
| **1 day** | 119.1 | 0.18 | 112.8 | 0.17 | 114.9 | 0.19 | 111.1 | 0.15 | 114.1 | 0.17 | 124.2 | 0.2 | 145.8 | 0.2 | 183.3 | 0.25 |
| **3 days** | *117.1* | *0.4* | 115.8 | 0.16 | 118.1 | 0.16 | 111.6 | 0.14 | 121.1 | 0.18 | 130 | 0.19 | 159.7 | 0.22 | 203.8 | 0.23 |
| **7 days** | *133.8* | *0.24* | 111.9 | 0.17 | 108.4 | 0.15 | 107 | 0.17 | 112.2 | 0.16 | 11.4 | 0.18 | 149.6 | 0.2 | 188.3 | 0.25 |
| **14 days** | | | 142.4 | 0.27 | 125.5 | 0.15 | 131.5 | 0.18 | 147.9 | 0.17 | 175.1 | 0.27 | 182.4 | 0.18 | 219 | 0.18 |
| **30 days** | | | | | | | | | | | | | | | | |

| L3 after sonication at 75% | | | | | | | | | | | | | | | | |
|---|---|---|---|---|---|---|---|---|---|---|---|---|---|---|---|---|
| **Chitosan concentration [%]** | **0.005** | | **0.01** | | **0.0175** | | **0.025** | | **0.05** | | **0.1** | | **0.175** | | **0.25** | |
| **TIME** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** | **Dim. [nm]** | **PDI** |
| **Time 0** | 120.1 | 0.19 | 107.8 | 0.15 | 112.2 | 0.15 | 107.7 | 0.18 | 118.1 | 0.19 | 122 | 0.2 | 140.3 | 0.23 | 183 | 0.24 |
| **1 day** | 122.3 | 0.22 | 117.7 | 0.21 | 114.6 | 0.17 | 114.1 | 0.16 | 117.9 | 0.18 | 127.2 | 0.19 | 161.6 | 0.21 | 188.9 | 0.23 |
| **3 days** | *121. 9* | *0.18* | 114.8 | 0.14 | 113.9 | 0.14 | 115.5 | 0.14 | 122.2 | 0.17 | 136.8 | 0.2 | 168.4 | 0.22 | 204.7 | 0.22 |
| **7 days** | | | 113.1 | 0.15 | 112.8 | 0.14 | 113.3 | 0.15 | 129.5 | 0.19 | 140.3 | 0.18 | 263.6 | 0.22 | 203 | 0.2 |
| **14 days** | | | | | | | | | | | | | | | | |
| **30 days** | | | | | | | | | | | | | | | | |

As can be seen from Table 4, even after stability tests at only one week, sonication improves the behaviour in the intermediate region of the concentrations; however, at the highest concentrations, which were those (according to the data of Table 3) which in the long run provide true stability, sonication is not effective, as shown by the large dimensions of the drops and the high PDI.

## Claims

1. A method for obtaining ultrastable nano-emulsions, comprising the steps of:
i) dispersing a first amount of a first fluid substance in a second amount, greater than the first amount, of a second fluid substance which is not miscible with the first fluid substance, in the presence of at least one ionic emulsifier preferably having chemical-physical affinities with the first fluid substance, so as to form a primary emulsion of drops having nanometric dimensions of the first fluid substance in a homogeneous phase defined by the second fluid substance and on the drops of which at least part of said at least one ionic emulsifier is adsorbed;
ii)- mixing the thus-formed primary emulsion in a solution of a polyelectrolyte in the second fluid substance, the polyelectrolyte being chosen so as to have an opposite polarity to that of the at least one ionic emulsifier, so as to form a secondary emulsion in which the drops of the first fluid substance are at least partially coated by a polymeric layer of polyelectrolyte; wherein it further comprises the steps of:
iii) standing the thus-obtained secondary emulsion; and
iv) processing the secondary emulsion so as to re-disperse the first fluid substance into the second fluid substance under the influence of a high shear stress;
**characterized in that**
step ii) is carried out in the presence of an over-concentration by weight of polyelectrolyte.

2. A method according to claim 1, **characterised in that** in step ii) the over-concentration by weight in polyelectrolyte in the second fluid substance is determined as follows:
- a plurality of secondary emulsion samples are prepared using increasing concentrations of polyelectrolyte;
- the theoretical concentration of polyelectrolyte necessary to saturate the primary emulsion (saturation concentration) is experimentally identified by means of a measurement of Z potential carried out on said secondary emulsion samples;
- step ii) is performed using a concentration of polyelectrolyte significantly greater than the saturation concentration determined by measurement of the Z potential on said samples.

3. A method according to claim 2, **characterised in that** in step ii) the concentration by weight in polyelectrolyte in the second fluid substance is chosen around one order of magnitude greater than the saturation concentration determined previously according to said measurement of Z potential.

4. A method according to any one of the preceding claims, **characterized in that** step i) is carried out by means of a high-pressure homogenizer operating at a first pressure, which is from 1000 to 3000 bar and, preferably, from 1500 to 2000 bar; the first and second fluid substances being passed into the homogenizer several times in succession, so as to carry out from 3 to 15 complete homogenization cycles or, alternatively, a plurality of cycles repeated in a continuous way or also combining complete (discrete) cycles and cycles carried out in a continuous way, measuring each time the average dimension obtained, until said average dimension reaches a minimum.

5. A method according to claim 4, **characterized in that** step iv) is carried out by means of a high-pressure homogenizer operating at a second pressure, which is however less than the first pressure at which step i) is carried out and which remains however from 200 to 1500 bar, and preferably from 500 to 1200 bar and, even more preferably, from 700 to 1000 bar; the first and second fluid substances being passed into the homogenizer several times in succession, so as to carry out at least 10 complete homogenization cycles or, alternatively, a plurality of cycles repeated in a continuous way or also combining complete (discrete) cycles and cycles carried out in a continuous way, measuring each time the polydispersity index, until the polydispersity (polydispersion) index reaches a minimum.

6. A method according to any one of the preceding claims, **characterized in that** said step iii), during which the secondary emulsion is left to stand, extends over a time interval from several hours to 1 month.

7. A method according to any one of the preceding claims, **characterized in that** said steps iii) and iv) are repeated at least once and, preferably, at least twice; the duration of step iii), prior to carrying out step iv) for the first time, being preferably 1 day; and the duration of step iii) prior to carrying out step iv) for the second time, being from one week to one month.

8. A method according to any one of the preceding claims, **characterized in that** the first fluid substance is a lipid substance and the second fluid substance is an aqueous substance; said ionic emulsifier being chosen in the group consisting of: soy lecithin, phosphatidylcholine (PC) and/or phosphatidylethanolamine (PE) enriched lecithin; and mixtures thereof; possibly, glycerol, pectin or other thickening agents being added to the second fluid substance.

9. A method according to claim 8, **characterized in that** said polyelectrolyte is chosen in the group consisting of biodegradable polymers.

10. A method according to any one of the preceding claims, **characterized in that** step i) is preceded by a pre-emulsion step in which said ionic emulsifier is progressively added to the first fluid substance operating under continuous mechanical mixing and in which the thus obtained mixture is sonicated; the subsequent step ii) being carried out by progressively adding the first fluid substance, with the addition of the ionic emulsifier, to a solution of polyelectrolyte in the second fluid substance operating under continuous mechanical mixing.

11. An ultrastable nano-emulsion comprising a first amount of a first fluid substance dispersed into a second amount, which is greater than the first amount, of a second fluid substance which is not miscible with the first fluid substance; the nano-emulsion further containing at least one ionic emulsifier adsorbed on respective drops of the first fluid substance dispersed into the second fluid substance and at least one polyelectrolyte having opposite polarity to that of the ionic emulsifier and which forms a polymeric coating around the drops of the first fluid substance; **characterized in that** it has been obtained according to the method of one of the claims from 1 to 10, so as to have a dimension of the drops of the first fluid substance coated by the ionic emulsifier and the polyelectrolyte of 50 to 300 nm; a dimensional distribution of the drops such that the nano-emulsion has a polydispersity index which is less than 0.1; and an over-time stability of said dimensional values of the drops of the first fluid substance which is longer than one year.

12. Nano-emulsion according to claim 11, **characterized in that** said drops of the first fluid substance dispersed into the second fluid substance present at least three coating layers including, starting from the inside outward: a first layer which forms a polymeric coating around the drops on which the ionic emulsifier is adsorbed and which is constituted by a polyelectrolyte having a polarity opposite to that of the ionic emulsifier; at least one second layer constituted by a polyelectrolyte having a polarity opposite to that of the polyelectrolyte forming the first layer; and at least a third layer preferably constituted by the same polyelctrolyte that forms the first layer.

13. Nano-emulsion according to claim 12, **characterized in that** said drops present from five, six and up to ten superimposed coating layers including compounds chosen in the group consisting in: chitosan, sodium alginate, heparin, polylysine and other polymers of comparable molecular weight and which are biodegradable.

## Patentansprüche

1. Verfahren zum Erlangen ultrastabiler Nanoemulsionen, das die Schritte aufweist:
i) Dispergieren einer ersten Menge einer ersten Fluidsubstanz in einer zweiten Menge, die größer als die erste Menge ist, einer zweiten Fluidsubstanz, die nicht mit der ersten Fluidsubstanz mischbar ist, in Gegenwart mindestens eines ionischen Emulgators, der vorzugsweise chemisch-physikalische Affinitäten mit der ersten Fluidsubstanz hat, um eine Primäremulsion aus Tropfen mit nanometrischen Abmessungen der ersten Fluidsubstanz in einer homogenen Phase zu bilden, die durch die zweite Fluidsubstanz definiert ist, und auf deren Tropfen mindestens ein Teil des mindestens einen ionischen Emulgators adsorbiert ist;
ii) Mischen der so gebildeten Primäremulsion in einer Lösung eines Polyelektrolyts in der zweiten Fluidsubstanz, wobei der Polyelektrolyt so gewählt ist, dass er eine entgegengesetzte Polarität zu der des mindestens einen ionischen Emulgators hat, um eine Sekundäremulsion zu bilden, in der die Tropfen der ersten Fluidsubstanz mindestens teilweise durch eine Polymerschicht aus Polyelektrolyt beschichtet sind; wobei es ferner die Schritte aufweist:
iii) Stehenlassen der so erhaltenen Sekundäremulsion; und
iv) Verarbeiten der Sekundäremulsion, um die erste Fluidsubstanz in der zweiten Fluidsubstanz unter dem Einfluss einer hohen Scherspannung erneut zu dispergieren;
**dadurch gekennzeichnet, dass**
der Schritt ii) in Gegenwart einer Gewichtsüberkonzentration von Polyelektrolyt durchgeführt wird.

2. Verfahren nach Anspruch 1, **dadurch gekennzeichnet, dass** im Schritt ii) die Gewichtsüberkonzentration von Polyelektrolyt in der zweiten Fluidsubstanz wie folgt bestimmt wird:
- mehrere Sekundäremulsionsproben werden unter Verwendung zunehmender Konzentrationen von Polyelektrolyt hergestellt;
- die theoretische Konzentration von Polyelektrolyt, die notwendig ist, um die Primäremulsion zu sättigen (Sättigungskonzentration), wird mit Hilfe einer Messung von Z-Potential experimentell identifiziert, die an den Sekundäremulsionsproben durchgeführt wird;
- der Schritt ii) wird unter Verwendung einer Konzentration von Polyelektrolyt durchgeführt, die erheblich größer als die Sättigungskonzentration ist, die durch Messung des Z-Potentials an den Proben bestimmt wird.

3. Verfahren nach Anspruch 2, **dadurch gekennzeichnet, dass** im Schritt ii) die Gewichtskonzentration von Polyelektrolyt in der zweiten Fluidsubstanz um rund eine Größenordnung größer als die Sättigungskonzentration gewählt wird, die zuvor gemäß der Messung des Z-Potentials bestimmt wurde.

4. Verfahren nach einem der vorstehenden Ansprüche, **dadurch gekennzeichnet, dass** der Schritt i) mit Hilfe eines Hochdruck-Homogenisators durchgeführt wird, der mit einem ersten Druck arbeitet, der 1000 bis 3000 bar und vorzugsweise 1500 bis 2000 bar beträgt; wobei die erste und zweite Fluidsubstanz mehrmals nacheinander in den Homogenisator geführt werden, um 3 bis 15 vollständige Homogenisationszyklen durchzuführen, oder alternativ mehrere Zyklen kontinuierlich wiederholt werden oder auch vollständige (diskrete) Zyklen und auf kontinuierliche Weise durchgeführte Zyklen kombiniert werden, wobei jedes Mal die erhaltene mittlere Abmessung gemessen wird, bis die mittlere Abmessung ein Minimum erreicht.

5. Verfahren nach Anspruch 4, **dadurch gekennzeichnet, dass** der Schritt iv) mit Hilfe eines Hochdruck-Homogenisators durchgeführt wird, der mit einem zweiten Druck arbeitet, der aber kleiner als der erste Druck ist, mit dem der Schritt i) durchgeführt wird, und der aber bei 200 bis 1500 bar und vorzugsweise 500 bis 1200 bar und noch stärker bevorzugt 700 bis 1000 bar bleibt; wobei die erste und zweite Fluidsubstanz mehrmals nacheinander in den Homogenisator geführt werden, um mindestens 10 vollständige Homogenisationszyklen durchzuführen, oder alternativ mehrere Zyklen kontinuierlich wiederholt werden oder auch vollständige (diskrete) Zyklen und auf kontinuierliche Weise durchgeführte Zyklen kombiniert werden, wobei jedes Mal der Polydispersitätsindex gemessen wird, bis der Polydispersitäts-(Polydispersions-) Index ein Minimum erreicht.

6. Verfahren nach einem der vorstehenden Ansprüche, **dadurch gekennzeichnet, dass** sich der Schritt iii), in dem die Sekundäremulsion stehen gelassen wird, über ein Zeitintervall von mehreren Stunden bis 1 Monat erstreckt.

7. Verfahren nach einem der vorstehenden Ansprüche, **dadurch gekennzeichnet, dass** die Schritte iii) und iv) mindestens einmal und vorzugsweise mindestens zweimal wiederholt werden; wobei die Dauer des Schritts iii) vor der erstmaligen Durchführung des Schritts iv) vorzugsweise 1 Tag beträgt; und die Dauer des Schritts iii) vor der zweitmaligen Durchführung des Schritts iv) eine Woche bis einen Monat beträgt.

8. Verfahren nach einem der vorstehenden Ansprüche, **dadurch gekennzeichnet, dass** die erste Fluidsubstanz eine Lipidsubstanz ist und die zweite Fluidsubstanz eine wässrige Substanz ist; wobei der ionische Emulgator aus der Gruppe ausgewählt ist, die aus Sojalecithin, Phosphatidylcholin (PC) und/oder mit Phosphatidylethanolamin (PE) angereichertem Lecithin; sowie deren Mischungen besteht; wobei eventuell Glycerol, Pektin oder andere Verdickungsmittel der zweiten Fluidsubstanz zugegeben sind.

9. Verfahren nach Anspruch 8, **dadurch gekennzeichnet, dass** der Polyelektrolyt aus der Gruppe ausgewählt ist, die aus biologisch abbaubaren Polymeren besteht.

10. Verfahren nach einem der vorstehenden Ansprüche, **dadurch gekennzeichnet, dass** dem Schritt i) ein Präemulsionsschritt vorausgeht, in dem der ionische Emulgator der ersten Fluidsubstanz unter kontinuierlichem mechanischem Mischen fortlaufend zugegeben wird und in dem die so erhaltene Mischung beschallt wird; wobei der anschließende Schritt ii) durch fortlaufendes Zugeben der ersten Fluidsubstanz mit der Zugabe des ionischen Emulgators zu einer Lösung von Polyelektrolyt in der zweiten Fluidsubstanz unter kontinuierlichem mechanischem Mischen durchgeführt wird.

11. Ultrastabile Nanoemulsion, die eine erste Menge einer ersten Fluidsubstanz aufweist, die in einer zweiten Menge, die größer als die erste Menge ist, einer zweiten Fluidsubstanz dispergiert ist, die nicht mit der ersten Fluidsubstanz mischbar ist; wobei die Nanoemulsion ferner mindestens einen ionischen Emulgator, der auf jeweiligen Tropfen der ersten Fluidsubstanz adsorbiert ist, die in der zweiten Fluidsubstanz dispergiert ist, und mindestens einen Polyelektrolyt enthält, der entgegengesetzte Polarität zu der des ionischen Emulgators hat und der eine Polymerbeschichtung um die Tropfen der ersten Fluidsubstanz bildet; **dadurch gekennzeichnet, dass** sie gemäß dem Verfahren nach einem der Ansprüche 1 bis 10 so erhalten wurde, dass sie eine Abmessung der Tropfen der ersten Fluidsubstanz, die durch den ionischen Emulgator und den Polyelektrolyt beschichtet sind, von 50 bis 300 nm hat; eine Abmessungsverteilung der Tropfen so hat, dass die Nanoemulsion einen Polydispersitätsindex hat, der kleiner als 0,1 ist; und eine zeitliche Stabilität der Abmessungswerte der Tropfen der ersten Fluidsubstanz hat, die länger als ein Jahr ist.

12. Nanoemulsion nach Anspruch 11, **dadurch gekennzeichnet, dass** die Tropfen der in der zweiten Fluidsubstanz dispergierten ersten Fluidsubstanz mindestens drei Beschichtungsschichten aufweisen, einschließlich von innen nach außen: einer ersten Schicht, die eine Polymerbeschichtung um die Tropfen bildet, auf denen der ionische Emulgator adsorbiert ist, und die durch einen Polyelektrolyt mit entgegengesetzter Polarität zu der des ionischen Emulgators gebildet ist; mindestens einer zweiten Schicht, die durch einen Polyelektrolyt gebildet ist, der eine entgegengesetzte Polarität zu der des Polyelektrolyts hat, der die erste Schicht bildet; und mindestens einer dritten Schicht, die vorzugsweise durch den gleichen Polyelektrolyt gebildet ist, der die erste Schicht bildet.

13. Nanoemulsion nach Anspruch 12, **dadurch gekennzeichnet, dass** die Tropfen fünf, sechs und bis zehn übereinander liegende Beschichtungsschichten haben, die Verbindungen aufweisen, die ausgewählt sind aus der Gruppe bestehend aus: Chitosan, Natriumalginat, Heparin, Polylysin und anderen Polymeren mit vergleichbarem Molekulargewicht, und die biologisch abbaubar sind.

## Revendications

1. Procédé d'obtention de nano-émulsions ultrastables, comprenant les étapes de :
i) dispersion d'une première quantité d'une première substance fluide dans une seconde quantité, plus grande que la première quantité, d'une seconde substance fluide qui n'est pas miscible avec la première substance fluide, en présence d'au moins un émulsifiant ionique ayant de préférence des affinités physico-chimiques avec la première substance fluide, de manière à former une émulsion primaire de gouttes ayant des dimensions nanométriques de la première substance fluide dans une phase homogène définie par la seconde substance fluide et sur les gouttes de laquelle au moins une partie dudit au moins un émulsifiant ionique est adsorbée ;
ii) mélange de l'émulsion primaire ainsi formée dans une solution d'un polyélectrolyte dans la seconde substance fluide, le polyélectrolyte étant choisi de manière à avoir une polarité opposée à celle du au moins un émulsifiant ionique, de manière à former une émulsion secondaire dans laquelle les gouttes de la première substance fluide sont au moins partiellement revêtues par une couche polymérique de polyélectrolyte ; dans lequel il comprend en outre les étapes de :
iii) laisser au repos l'émulsion secondaire ainsi obtenue ; et
iv) traitement de l'émulsion secondaire de manière à redisperser la première substance fluide dans la seconde substance fluide sous l'influence d'une forte contrainte de cisaillement ;
**caractérisé en ce que**
l'étape ii) est réalisée en présence d'une surconcentration en poids de polyélectrolyte.

2. Procédé selon la revendication 1, **caractérisé en ce que** dans l'étape ii), la surconcentration en poids en polyélectrolyte dans la seconde substance fluide est déterminée comme suit :
- une pluralité d'échantillons de l'émulsion secondaire sont préparés en utilisant des concentrations croissantes en polyélectrolyte ;
- la concentration théorique en polyélectrolyte nécessaire pour saturer l'émulsion primaire (concentration à saturation) est identifiée expérimentalement au moyen d'une mesure de potentiel Z réalisée sur lesdits échantillons d'émulsion secondaire ;
- l'étape ii) est réalisée en utilisant une concentration en polyélectrolyte significativement plus grande que la concentration à saturation déterminée par une mesure du potentiel Z sur lesdits échantillons.

3. Procédé selon la revendication 2, **caractérisé en ce que** dans l'étape ii), la concentration en poids en polyélectrolyte dans la seconde substance fluide est choisie à environ un ordre de grandeur plus grand que la concentration à saturation déterminée au préalable selon ladite mesure de potentiel Z.

4. Procédé selon l'une quelconque des revendications précédentes, **caractérisé en ce que** l'étape i) est réalisée au moyen d'un homogénéisateur haute pression fonctionnant à une première pression, qui est de 1 000 à 3 000 bars et, de préférence, de 1 500 à 2 000 bars ; en faisant passer les première et seconde substances fluides plusieurs fois dans l'homogénéisateur en succession, de manière à réaliser de 3 à 15 cycles d'homogénéisation complets ou, alternativement, une pluralité de cycles répétés de manière continue ou en combinant également des cycles complets (discrets) et des cycles réalisés de manière continue, en mesurant à chaque fois la dimension moyenne obtenue, jusqu'à ce que ladite dimension moyenne atteigne un minimum.

5. Procédé selon la revendication 4, **caractérisé en ce que** l'étape iv) est réalisée au moyen d'un homogénéisateur haute pression fonctionnant à une seconde pression, qui est néanmoins inférieure à la première pression à laquelle l'étape i) est réalisée et qui reste néanmoins de 200 à 1 500 bars et, de préférence de 500 à 1 200 bars et, de manière encore davantage préférée, de 700 à 1 000 bars ; en faisant passer les première et seconde substances fluides plusieurs fois dans l'homogénéisateur en succession, de manière à réaliser au moins 10 cycles d'homogénéisation complets ou, alternativement, une pluralité de cycles répétés de manière continue ou en combinant également des cycles complets (discrets) et des cycles réalisés de manière continue, en mesurant à chaque fois l'indice de polydispersité, jusqu'à ce que l'indice de polydispersité (polydispersion) atteigne un minimum.

6. Procédé selon l'une quelconque des revendications précédentes, **caractérisé en ce que** ladite étape iii), pendant laquelle l'émulsion secondaire est laissée au repos, s'étend sur un intervalle de temps allant de plusieurs heures à 1 mois.

7. Procédé selon l'une quelconque des revendications précédentes, **caractérisé en ce que** lesdites étapes iii) et iv) sont répétées au moins une fois et, de préférence, au moins deux fois ; la durée de l'étape iii), avant de réaliser l'étape iv) pour la première fois, étant de préférence de 1 jour ; et la durée de l'étape iii) avant de réaliser l'étape iv) pour la seconde fois, étant de une semaine à un mois.

8. Procédé selon l'une quelconque des revendications précédentes, **caractérisé en ce que** la première substance fluide est une substance lipidique et la seconde substance fluide est une substance aqueuse ; ledit émulsifiant ionique étant choisi dans le groupe constitué par : la lécithine de soja, la phosphatidylcholine (PC) et/ou la lécithine enrichie en phosphatidyléthanolamine (PE) ; et leurs mélanges ; éventuellement, du glycérol, de la pectine ou d'autres agents épaississants étant ajoutés à la seconde substance fluide.

9. Procédé selon la revendication 8, **caractérisé en ce que** ledit polyélectrolyte est choisi dans le groupe constitué par les polymères biodégradables.

10. Procédé selon l'une quelconque des revendications précédentes, **caractérisé en ce que** l'étape i) est précédée par une étape de pré-émulsion dans laquelle ledit émulsifiant ionique est progressivement ajouté à la première substance fluide sous un mélange mécanique continu et dans lequel le mélange ainsi obtenu fait l'objet d'une sonication ; l'étape ii) ultérieure étant réalisée en ajoutant progressivement la première substance fluide, avec l'addition de l'émulsifiant ionique, à une solution de polyélectrolyte dans la seconde substance fluide sous un mélange mécanique continu.

11. Nano-émulsion ultrastable comprenant une première quantité d'une première substance fluide dispersée dans une seconde quantité, qui est plus grande que la première quantité, d'une seconde substance fluide qui n'est pas miscible avec la première substance fluide ; la nano-émulsion contenant en outre au moins un émulsifiant ionique adsorbé sur des gouttes respectives de la première substance fluide dispersées dans la seconde substance fluide et au moins un polyélectrolyte ayant une polarité opposée à celle de l'émulsifiant ionique, et qui forme un revêtement polymérique autour des gouttes de la première substance fluide ; **caractérisée en ce qu'**elle a été obtenue selon le procédé de l'une des revendications 1 à 10, de manière à avoir une dimension des gouttes de la première substance fluide revêtue par l'émulsifiant ionique et le polyélectrolyte de 50 à 300 nm ; une distribution dimensionnelle des gouttes telle que la nano-émulsion ait un indice de polydispersité qui est de moins de 0,1 ; et une stabilité au cours du temps desdites valeurs dimensionnelles des gouttes de la première substance fluide qui est plus longue qu'une année.

12. Nano-émulsion selon la revendication 11, **caractérisée en ce que** lesdites gouttes de la première substance fluide dispersées dans la seconde substance fluide présentent au moins trois couches de revêtement comprenant, en partant de l'intérieur vers l'extérieur : une première couche qui forme un revêtement polymérique autour des gouttes sur lesquelles l'émulsifiant ionique est adsorbé et qui est constituée par un polyélectrolyte ayant une polarité opposée à celle de l'émulsifiant ionique ; au moins une deuxième couche constituée par un polyélectrolyte ayant une polarité opposée à celle du polyélectrolyte formant la première couche ; et au moins une troisième couche de préférence constituée par le même polyélectrolyte que celui qui forme la première couche.

13. Nano-émulsion selon la revendication 12, **caractérisée en ce que** lesdites gouttes présentent de cinq, six et jusqu'à dix couches de revêtement superposées comprenant des composés choisis dans le groupe constitué par : le chitosane, l'alginate de sodium, l'héparine, la polylysine et d'autres polymères de masse moléculaire comparable et qui sont biodégradables.
